(19) Europäisches Patentamt
European Patent Office
Office européen des brevets

(11) Numéro de publication: **0 095 408**
**B1**

(12) **FASCICULE DE BREVET EUROPEEN**

(45) Date de publication du fascicule du brevet: **24.07.85**

(51) Int. Cl.⁴: **C 07 C 29/136**, C 07 C 31/08, C 07 C 31/12, C 07 C 31/125

(21) Numéro de dépôt: **83400953.2**

(22) Date de dépôt: **11.05.83**

(54) **Procédé catalytique de production d'alcools par hydrogénolyse d'esters d'acides carboxyliques.**

(30) Priorité: **24.05.82 FR 8209099**

(43) Date de publication de la demande: **30.11.83 Bulletin 83/48**

(45) Mention de la délivrance du brevet: **24.07.85 Bulletin 85/30**

(84) Etats contractants désignés: **BE CH DE GB IT LI NL**

(56) Documents cités:
**DE - A - 3 019 582**
**US - A - 3 825 503**

**CHEMICAL ABSTRACTS, vol. 93, no. 25, 22 decembre 1980, page 761, no. 238371s, Columbus, Ohio, USA YU.I. ERMAKOV et al.: "Modification of catalytic proporties of silica-supported rhodium"**

(73) Titulaire: **INSTITUT FRANCAIS DU PETROLE, 4, Avenue de Bois-Préau, F-92502 Rueil-Malmaison (FR)**

(72) Inventeur: **Travers, Christine, 15 ter, rue Beaumarchais, F-92500 Rueil-Malmaison (FR)**
Inventeur: **Trinh Dinh, Chan, 7, Allée des Oeillets, F-78110 Le Vésinet (FR)**
Inventeur: **Snappe, Roger, 32, rue des Hauts Closeaux, F-92310 Sèvres (FR)**
Inventeur: **Bournonville, Jean-Paul, 5, rue des Champs Rogers, F-78400 Chatou (FR)**

(74) Mandataire: **Colas des Francs, Jean et al, Institut Français du Pétrole 4, Avenue de Bois Préau, F-92502 Rueil-Malmaison (FR)**

**EP 0 095 408 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

L'invention concerne un procédé catalytique de production d'alcools par hydrogénolyse d'esters d'acides carboxyliques.

La production d'alcools, et en particulier d'alcools gras, présente un intérêt considérable pour l'industrie.

L'hydrogénolyse catalytique des esters d'acides carboxyliques représente une voie intéressante de production de ces alcools mais elle s'est trouvée freinée jusqu'à présent par les inconvénients des catalyseurs connus:

— les catalyseurs à base d'oxydes mixtes de cuivre et de chrome, dopés ou non, nécessitent de travailler sous pression élevée, dans la quasi-totalité des cas supérieure à 200 atmosphères, et à une température comprise entre 250 et 350° C,

— les catalyseurs à base de métaux de transition déposés sur un support obligent à travailler à une température moins élevée, inférieure à 250° C et de préférence à 200° C, pour limiter la dégradation de l'alcool formé en hydrocarbures; ce qui impose des pressions opératoires supérieures à 100 bar pour obtenir de bonnes sélectivités à un niveau de conversion acceptable.

Il a été découvert, de façon surprenante, qu'il est possible de réaliser l'hydrogénation d'un ester en alcool sans modification de la structure de la chaîne hydrocarbonnée, selon le schéma suivant:

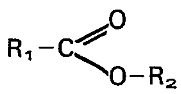 $+ 2H_2 \rightarrow R_1CH_2OH + R_2OH$

où $R_1$ = radical hydrocarbyl, saturé ou insaturé, de 1-20 atomes de carbone et $R_2$ = alkyl de 1-10 atomes de carbone, de préférence méthyl ou éthyl. On opère de préférence dans un réacteur continu ou discontinu sous une pression totale comprise entre 10 et 100 bar et de préférence entre 30 et 80 bar, bien que l'on puisse opérer sans inconvénient, par exemple, jusqu'à 300 bar, à une température comprise entre 180 et 330° C et de préférence entre 200 et 280° C et avec un rapport molaire hydrogène sur ester compris entre 2 et 10 et plus avantageusement entre 2 et 5, en présence d'un catalyseur métallique supporté renfermant les éléments suivants: le rhodium dont le pourcentage pondéral est choisi entre 0,1 et 5% et de préférence entre 0,5 et 2% et au moins un élément choisi dans le groupe constitué par le germanium, l'étain et le plomb et dont le pourcentage pondéral est compris entre 0,1 et 10% et plus particulièrement entre 2 et 5%. On peut avantageusement utiliser à la fois deux des métaux ci-dessus ou même ces trois métaux; le support peut être choisi dans le groupe constitué par la silice, les différents types d'alumine, les silices alumines, le charbon et de préférence dans le groupe constitué par les différents types d'alumine. L'utilisation de support d'alumine contenant de 1 à 5% d'alcalin (Na, K) ou d'alcalino-terreux (Mg, Ca, Ba) peut également être envisagée.

Le catalyseur peut être préparé par différentes procédures d'imprégnation du support et l'invention n'est pas limitée à une procédure déterminée. L'opération d'imprégnation consiste, par exemple, à mettre en contact le support préformé et une solution aqueuse ou organique d'un composé du métal ou des métaux choisi(s), le volume de solution étant en excès par rapport au volume de rétention du support ou égal à ce volume. On peut introduire le rhodium et le métal additionnel simultanément ou successivement. Après avoir laissé le contact entre le support et la solution pendant plusieurs heures, le support imprégné est filtré, lavé à l'eau distillée, séché et calciné sous air entre 110 et 600° C et de préférence entre 110 et 500° C. Avant utilisation, on réduit le catalyseur sous hydrogène entre 200 et 600° C et de préférence entre 300 et 500° C, cette réduction pouvant être effectuée aussitôt après la calcination, ou plus tard, chez l'utilisateur.

L'élément choisi dans le groupe constitué par l'étain, le germanium et le plomb peut être introduit en solution aqueuse ou en solution hydrocarbonée selon la nature du précurseur utilisé. On préfère introduire d'abord le rhodium et calciner ou calciner et réduire, dans des conditions indiquées plus haut et introduire ensuite l'étain, le germanium et/ou le plomb.

Une autre méthode consiste à malaxer la poudre humide de support avec les précurseurs du catalyseur et à mettre ensuite en forme et sécher.

Les exemples de précurseurs métalliques utilisables dans la préparation du catalyseur sont les suivants:

Pour le rhodium, on peut employer des composés tels que les chlorures, nitrates ou sels d'acides organiques solubles dans le solvant d'imprégnation, par exemple le chlorure de rhodium, le nitrate de rhodium, les sels solubles d'acides organiques comme l'acétate de rhodium, mais également les chlorure ou nitrate de rhodium hexamine. On peut encore utiliser des composés organométalliques de rhodium en solution dans un hydrocarbure, par exemple dans un hydrocarbure paraffinique saturé dont la chaîne hydrocarbonée contient de 6 à 12 atomes de carbone, dans un hydrocarbure naphténique qui contient de 6 à 12 atomes de carbone ou dans un hydrocarbure aromatique contenant de 6 à 11 atomes de carbone; on utilisera de préférence l'acétylacétonate de rhodium.

L'élément choisi dans le groupe constitué de l'étain, du germanium et du plomb peut être introduit par l'intermédiaire de composés tels que les chlorures, les bromures et le nitrate d'étain, les halogénures, nitrate, acétate et carbonate de plomb, le chlorure et l'oxalate de germanium en solution aqueuse ou, de préférence, sous forme des alkyl ou des arylmétaux d'étain, de germanium et de plomb tels que par exemple: le tétrabutyl-étain, le tétraméthyl-étain, le tétrapropyl-germanium, le tétraéthyl-plomb, le diphényl-étain, le diphényl-germanium, le tétraphényl-plomb en solution hydrocarbonée.

Le support peut être de nature variée, comme déjà mentionné plus haut. Un support particulièrement adapté possède des caractéristiques spécifiques telles qu'une surface spécifique, déterminée par la méthode B.E.T., comprise entre 10 et

500 mètres carrés par gramme et de préférence comprise entre 50 et 500 mètres carrés par gramme et un volume poreux total compris entre 20 et 130 $cm^3$ pour 100 grammes de support et de préférence compris entre 50 et 110 $cm^3$ pour 100 grammes de support.

Une fois les deux métaux fixés sur le support, le catalyseur subit avantageusement un traitement d'activation sous hydrogène à haute température, par exemple 300-500° C, afin d'obtenir une phase métallique active. La procédure de ce traitement sous hydrogène consiste par exemple en une montée lente de la température sous courant d'hydrogène jusqu'à la température maximale de réduction, comprise par exemple entre 300 et 500° C et de préférence entre 350 et 450° C, suivie d'un maintien pendant 1 à 6 heures à cette température.

Les exemples suivants, non limitatifs, illustrent l'invention.

*Exemple 1*

La préparation du catalyseur se fait en deux étapes:

– fixation du rhodium par imprégnation de trichlorure de rhodium en solution aqueuse sur une alumine dont la surface spécifique est égale à 200 $m^2$ par gramme et le volume poreux total est égal à 60 $cm^3$ pour 100 grammes, suivie d'une filtration, d'un séchage à 110° C, d'une calcination sous air à 450° C et d'une réduction sous hydrogène à 450° C,

– fixation de l'étain sur le support préimprégné par le rhodium, calciné et réduit, sous forme de tétraéthyl-étain en solution dans le normal-heptane. Après avoir laissé en contact pendant 4 heures le catalyseur et la solution de tétraéthyl-étain au reflux de l'heptane, le catalyseur est lavé par l'heptane puis séché.

Le catalyseur est ensuite chargé dans un réacteur tubulaire puis réduit pendant 4 heures à 300° C sous courant d'hydrogène.

Les conditions opératoires pour l'hydrogénolyse de l'acétate d'éthyle sont les suivantes:

– pression: 50 bar
– VVH: 4 l/l de catalyseur/h
– rapport molaire $H_2$/ester: 5.

Dans cette première série d'essais on a fait varier la teneur en étain des catalyseurs en partant d'un catalyseur de base contenant 1% en poids de rhodium. La température de travail a été fixée à 250° C. Les résultats sont donnés dans le tableau 1.

*Tableau 1*

| Rh (% pds) | Sn (% pds) | Conversion totale (% pds) | Rendement (éthanol) (% pds) |
|---|---|---|---|
| 1 | 0 | 92 | 2,0 |
| 1 | 0,5 | 3 | 2,2 |
| 1 | 0,8 | 6 | 5,5 |
| 1 | 1,5 | 13,6 | 12,8 |
| 1 | 2,3 | 25,6 | 22,1 |
| 1 | 3,2 | 31,6 | 30,7 |

*Exemple 2*

On a comparé, à différentes températures, les propriétés catalytiques, vis-à-vis de l'hydrogénolyse de l'acétate d'éthyle, de deux catalyseurs: l'un à base de 1% de rhodium sur l'alumine de l'exemple 1, l'autre à base de 1% de rhodium et 3,2% d'étain sur le même support d'alumine. Toutes les autres conditions sont identiques à celles de l'exemple 1.

Les résultats figurent dans le tableau 2.

*Tableau 2*

| Température (° C) | Catalyseur | | | |
|---|---|---|---|---|
| | 1% Rh/$Al_2O_3$ | | 1% Rh + 3,2% Sn / $Al_2O_3$ | |
| | Conversion (% pds) | Rendement (éthanol) (% pds) | Conversion (% pds) | Rendement (éthanol) (% pds) |
| 200 | 2,6 | 1,7 | 6,6 | 6,5 |
| 220 | 19,4 | 6,0 | 16,0 | 15,8 |
| 250 | 92 | 2,0 | 31,6 | 30,7 |
| 280 | 100 | 0 | 70,7 | 66,3 |

Dans tout l'intervalle de température considéré, on observe que le catalyseur bimétallique rhodium-étain déposé sur alumine est incomparablement plus sélectif pour la production d'alcool.

*Exemple 3*

On se propose de fabriquer de l'éthanol à partir d'acétate d'éthyle dans des conditions identiques à celles adoptées dans l'exemple 1. Le catalyseur est à base de rhodium (1%) déposé sur l'alumine de l'exemple 1 et d'un deuxième élément du groupe de l'étain du germanium et du plomb.

Le germanium et le plomb sont imprégnés en solution hydrocarbonée (normal-heptane) respectivement sous forme de tétraéthyl-germanium et de tétraéthyl-plomb.

Les catalyseurs, ainsi préparés, sont mis en œuvre de la même façon que dans l'exemple 1 (T: 250° C, P: 50 bar, VVH = 4, rapport molaire $H_2$/ester: 5).

Les résultats sont donnés dans le tableau 3.

*Tableau 3*

| Rh (% pds) | 2e métal (% pds) | Conversion (% pds) | Rendement (éthanol) (% pds) |
|---|---|---|---|
| 1 | 0 | 92 | 2,0 |
| 1 | Sn = 3,2 | 31,6 | 30,7 |
| 1 | Ge = 3,1 | 30,9 | 30,5 |
| 1 | Pb = 3,4 | 31,9 | 30,6 |

L'étain peut donc être remplacé par le germanium et le plomb sans altération significative des propriétés catalytiques de la masse active.

*Exemple 4*

On prépare, selon la méthode décrite dans l'exemple 1, des catalyseurs à base de rhodium et d'étain sur une silice dont la surface spécifique est égale à 450 $m^2$ par gramme et le volume poreux total à 80 $cm^3$ pour 100 grammes. L'hydrogénolyse de l'acétate d'éthyle a été effectuée dans des conditions identiques à celles de l'exemple 1.

Les résultats sont donnés dans le tableau 4.

*Tableau 4*

| Rhodium (% pds) | Etain (% pds) | Conversion (% pds) | Rendement (éthanol) (% pds) | Sélectivité (éthanol) (% pds) |
|---|---|---|---|---|
| 1 | 0 | 7 | 2,2 | 31,5 |
| 1 | 0,8 | 8,6 | 8,4 | 97,7 |
| 1 | 1,5 | 17,4 | 17,0 | 97,7 |
| 1 | 3,0 | 11,1 | 10,7 | 96,3 |

On obtient donc des sélectivités de transformation en alcool très élevées, dans tous les cas supérieures à 95%.

*Exemple 5*

On se propose de fabriquer différents alcools à partir de divers esters en présence d'un catalyseur (1% Rh + 3,2% Sn sur alumine + 1,5% K) et dans des conditions opératoires identiques à celles de l'exemple 1.

Les esters utilisés sont les suivants:

- acétate de butyle secondaire,
- acétate d'amyle,
- acétate d'hexyle,
- caprate d'éthyle,
- palmitate de méthyle,
- oléate de méthyle.

Les résultats sont groupés dans le tableau 5.

*Tableau 5*

| Substrat | Alcools obtenus | Conversion (% pds) | Rendement alcools (% pds) |
|---|---|---|---|
| Acétate de butyle secondaire | Ethanol et butanol-2 | 30,5 | 29,0 |
| Acétate d'isoamyle | Ethanol et alcool isoamylique | 33,0 | 31,5 |
| Acétate d'hexyle | Ethanol et hexanol-1 | 32,0 | 30,0 |
| Caprate d'éthyle | Décanol-1 et éthanol | 35,0 | 33,1 |
| Palmitate de méthyle | Hexadécanol-1 et méthanol | 32 | 30,5 |
| Oléate de méthyle | Octadécanol-1 et méthanol | 34 | 32,8 |

## Revendications

1. Procédé de fabrication d'alcools dans lequel un ester d'acide carboxylique est traité par l'hydrogène en présence d'un catalyseur, caractérisé en ce que le catalyseur renferme un support, du rhodium et au moins un second élément choisi dans le groupe constitué de l'étain, du germanium et du plomb.

2. Procédé selon la revendication 1, dans lequel le catalyseur a une teneur en rhodium de 0,1 à 5% en poids et une teneur en second élément de 0,1 à 10% en poids.

3. Procédé selon la revendication 1 ou 2, dans lequel le support présente une surface de 10 à 500 $m^2/g$ et un volume poreux de 0,2 à 1,3 $cm^3/g$.

4. Procédé selon l'une des revendications 1 à 3, dans lequel le support est l'alumine.

5. Procédé selon l'une des revendications 1 à 3, dans lequel le support est la silice.

6. Procédé selon l'une des revendications 1 à 5, dans lequel la pression est de 10 à 100 bar et la température de 180 à 330° C.

7. Procédé selon l'une des revendications 1 à 6, dans lequel le rapport molaire hydrogène/ester est de 2: 1 à 10: 1.

8. Catalyseur utilisable dans la mise en œuvre du procédé selon l'une des revendications 1 à 7, caractérisé en ce qu'il résulte de la mise en contact d'un support avec un composé de rhodium et avec au moins un composé choisi parmi les alkyl- et aryl- métaux d'étain, de germanium et de plomb.

9. Catalyseur selon la revendication 8, caractérisé en ce qu'il résulte de la mise en contact d'un support avec une solution d'un composé du rhodium, séchage et calcination à une température comprise entre 110 et 600° C puis mise en contact avec une solution dans un solvant hydrocarboné, d'au moins un composé choisi parmi les alkyl- et aryl- métaux d'étain, de germanium et de plomb.

10. Catalyseur selon la revendication 9, caractérisé en ce qu'après la calcination à 110-600° C, on effectue une réduction par l'hydrogène à 200-600° C avant la mise en contact avec ladite solution hydrocarbonée, une activation finale par l'hydrogène à 300-500° C étant effectuée sur le produit contenant lesdits alkyl- et aryl- métaux.

## Patentansprüche

1. Verfahren zur Herstellung von Alkoholen, bei dem ein Ester einer Carbonsäure mit Wasserstoff in Gegenwart eines Katalysators behandelt wird, dadurch gekennzeichnet, dass man einen Katalysator einsetzt, der einen Träger, Rhodium und mindestens ein zweites Element, bestehend aus Zinn, Germanium oder Blei, aufweist.

2. Verfahren nach Anspruch 1, dadurch gekennzeichnet, dass der Katalysator einen Rhodiumgehalt von 0,1 bis 5 Gewichtsprozent und einen Gehalt an einem Zweitelement von 0,1 bis 10 Gewichtsprozent aufweist.

3. Verfahren nach den Ansprüchen 1 oder 2, dadurch gekennzeichnet, dass der Träger eine spezifische Oberfläche von 10 bis 500 $m^2/g$ und ein Porenvolumen von 0,2 bis 1,3 $cm^3/g$ aufweist.

4. Verfahren nach einem der Ansprüche 1 bis 3, dadurch gekennzeichnet, dass der Träger aus Aluminiumoxid besteht.

5. Verfahren nach einem der Ansprüche 1 bis 3, dadurch gekennzeichnet, dass der Träger aus Siliziumdioxid besteht.

6. Verfahren nach einem der Ansprüche 1 bis 5, dadurch gekennzeichnet, dass man einen Druck von 10 bis 100 bar und eine Temperatur von 180 bis 330° C anwendet.

7. Verfahren nach einem der Ansprüche 1 bis 6, dadurch gekennzeichnet, dass man bei einem molaren Verhältnis Wasserstoff/Ester von 2: 1 bis 10: 1 arbeitet.

8. Katalysator, der zur Durchführung des Verfahrens nach einem der Ansprüche 1 bis 7 geeignet ist, dadurch gekennzeichnet, dass er gewonnen ist durch Kontaktieren eines Trägers mit einer Rhodiumverbindung und mit mindestens einer Metallalkyl- oder -arylverbindung von Zinn, Germanium oder Blei.

9. Katalysator nach Anspruch 8, dadurch gekennzeichnet, dass er gewonnen ist durch Kontaktieren eines Trägers mit einer Lösung einer Rhodiumverbindung, Trocknen und Kalzinieren bei einer Temperatur zwischen 110 und 600° C und anschliessendes Kontaktieren mit einer Lösung mindestens einer Metallalkyl- oder -arylverbindung von Zinn, Germanium oder Blei in einem Kohlenwasserstofflösungsmittel.

10. Katalysator nach Anspruch 9, dadurch gekennzeichnet, dass nach dem Kalzinieren bei 110 bis 600° C eine Reduktion mit Wasserstoff bei 200 bis 600° C vor dem Kontaktieren mit der Kohlenwasserstofflösung erfolgt ist und das metallalkyl- oder -arylhaltige Produkt einer abschliessenden Aktivierung mit Wasserstoff bei 300 bis 500° C unterworfen wurde.

## Claims

1. A process for manufacturing alcohols, wherein an ester of a carboxylic acid is treated with hydrogen in the presence of a catalyst, characterized in that the catalyst comprises a carrier, rhodium and at least one second element selected from the group consisting of tin, germanium and lead.

2. A process according to claim 1, wherein the catalyst has a rhodium content of 0.1 to 5% b.w. and a content of second element from 0.1 to 10% b.w.

3. A process according to claims 1 or 2, wherein the carrier has a surface of 10 to 500 $m^2/g$ and a pore volume of 0.2 to 1.3 cc/g.

4. A process according to one of claims 1 to 3, wherein the carrier is alumina.

5. A process according to one of claims 1 to 3, wherein the carrier is silica.

6. A process according to one of claims 1 to 5, wherein the pressure is from 10 to 100 bar and the temperature from 180 to 330° C.

7. A process according to one of claims 1 to 6, wherein the molar ratio hydrogen/ester is from 2:1 to 10: 1.

8. Catalyst to be used in a process according to one of claims 1 to 7, characterized in that the catalyst is made by contacting a carrier with a rhodium compound and with at least one metal compound selected from the alkyl- and aryl- tin, germanium and lead compounds.

9. Catalyst according to claim 8, wherein said contacting is performed by contacting the carrier with a solution of said rhodium compound, drying and calcining at a temperature between 110° C and 600° C, and then contacting with a hydrocarbon solution of at least one metal compound selected from the alkyl- and aryl- tin, germanium and lead compounds.

10. Catalyst according to claim 9, characterized in that after the calcination at 110-600° C, a reduction with hydrogen at 200-600° C is done before

the contact with said hydrocarbon solution, and a final activation with hydrogen at 300-500°C is done on the product containing said alkyl- and aryl- metal compounds.